# EUROPÄISCHE PATENTANMELDUNG

(11) **EP 3 262 931 A1**
(43) Veröffentlichungstag der Anmeldung: **03.01.2018**
(21) Anmeldenummer: 17175326.2
(22) Anmeldetag: 09.06.2017
(51) Int. Cl.: A01K 31/04, A01K 31/00, A01K 1/01

(54) **TROCKNUNGSVORRICHTUNG ZUR TROCKNUNG EINER SUBSTANZ, GEFLÜGELHALTESYSTEM SOWIE VERFAHREN ZUR TROCKNUNG EINER SUBSTANZ**

(30) Priorität: 27.06.2016 DE 202016103370 U
(71) Anmelder: Big Dutchman International GmbH, 49377 Vechta (DE)
(72) Erfinder: THEMANN, Ludger, 49377 Vechta (DE)
(74) Vertreter: Eisenführ Speiser

(57) **Zusammenfassung**

Die Erfindung betrifft eine Trocknungsvorrichtung (100) zur Trocknung einer Substanz, insbesondere zur Trocknung von Geflügelkot, umfassend eine Trocknungseinheit (110) mit einer Fördervorrichtung (100), einen Trockensubstanzsensor (410) und einen Ammoniaksensor (420).

## Beschreibung

Die Erfindung betrifft eine Trocknungsvorrichtung zur Trocknung einer Substanz, ein Steuerungssystem für eine Trocknungsvorrichtung zum Trocknen einer Substanz, ein Trocknungssystem, ein Geflügelhaltungssystem und ein Verfahren zur Trocknung einer Substanz.

Landwirtschaftliche Nutztiere, beispielsweise Geflügeltiere, wie Legehennen, Hähnchen, Broiler, Puten oder Enten werden in der Regel in landwirtschaftlichen Betrieben gehalten, wobei insbesondere Stallanlagen eingesetzt werden. In diesen landwirtschaftlichen Betrieben, insbesondere in den Stallanlagen der landwirtschaftlichen Betriebe, werden tierische Produkte erzeugt. In modernen, hochtechnisierten Stallanlagen werden die Umgebungsbedingungen der Tiere optimiert, um beispielsweise eine artgerechte Tierhaltung und gleichzeitig eine effiziente Produktion zu ermöglichen. Die Umgebungsbedingungen betreffen beispielsweise die im Stall befindliche Luft und/oder die Bedingungen auf dem Stallboden. Unter tierischen Produkten werden im Folgenden insbesondere die Nebenprodukte der eigentlichen Produktion verstanden, wie beispielsweise Tierkot, insbesondere Geflügelkot und/oder Federn, die beispielsweise bei der Aufzucht, Eierproduktion oder Fleischproduktion entstehen.

Ein Bestandteil moderner Stallanlagen sind Fördervorrichtungen zum Fördern von tierischen Produkten. Die Fördervorrichtungen können beispielsweise als Bandförderer (auch als Gurtförderer bezeichnet) oder Plattenförderer ausgebildet sein. Ein Bandförderer umfasst in der Regel ein Förderband, meist ein Endlosförderband, und einen Bandantrieb, der das Förderband in einer oder mehreren Förderrichtungen antreibt. Anstelle eines Bandförderers kann auch ein Plattenförderer vorgesehen sein, der sich von einem Bandförderer im Wesentlichen dadurch unterscheidet, dass anstelle des Förderbands eine Reihe von verbundenen Platten eingesetzt wird. Aufgrund der Ähnlichkeit von Band- und Plattenförderern in Bezug auf die hier vorliegende Thematik, sollen dann, wenn im Folgenden von Bandförderern und Förderbändern gesprochen wird, Plattenförderer und Förderplatten entsprechend mit umfasst sein. Fördervorrichtungen dienen insbesondere dazu, aus verschiedenen Tierhaltungssystemen tierische Produkte schnell und zuverlässig ab bzw. weiter zu transportieren. Bei Fördervorrichtungen zum Fördern von zu trocknenden Substanzen, wie beispielsweise Geflügelkot, kann zusätzlich vorgesehen sein, die Substanz zu belüften, um diese zu trocknen oder in separaten Trocknungsvorrichtungen zu trocknen, in denen ebenfalls die im Vorherigen beschriebenen Fördervorrichtungen eingesetzt werden können.

Die Trocknung der Nebenprodukte, insbesondere des Geflügelkots, ist die Voraussetzung dafür, dass diese gut zu lagern und weiter zu vermarkten sind. Für die Trocknung werden vorzugsweise Trocknungstunnel verwendet, in denen Luft, beispielsweise die warme Abluft aus einem Stall, über Ventilatoren in einen Druckkorridor geleitet wird. Über und neben dem Druckkorridor sind vorzugsweise mehrere Etagen perforierter Förderbänder, auch als Trocknungsbänder bezeichnet, angeordnet, sodass die Abluft durch Öffnungen in den Trocknungstunnel einströmen und durch die Perforation hindurch strömen kann, wodurch dem Gut Feuchtigkeit entzogen und das Gut getrocknet werden kann. Die Trocknung kann in einem geschlossenen Raum stattfinden, wenn die vom Trocknungstunnel abgegebene Luft gefiltert werden soll, wodurch die Möglichkeit geschaffen wird, den Trocknungsprozess weitgehend geruchsneutral zu gestalten. Voraussetzung für eine optimale Trocknung ist, dass die Bänder möglichst gleichmäßig mit dem zu trocknenden Gut beladen werden.

Eine gleichmäßige Trocknung des zu trocknenden Guts, insbesondere des Geflügelkots, hat vorteilhafte Auswirkungen auf die Lagerfähigkeit und die Vermarktung der Substanz. Insbesondere könnten somit Folgekosten vermindert werden. Insbesondere wird eine gleichmäßig getrocknete Substanz, insbesondere Geflügelkot, die vorzugsweise einen hohen Trockensubstanzanteil aufweist, als hochwertiger angesehen. Die Trocknung der Substanz wird durch unterschiedliche Parameter beeinflusst. Neben der Höhe der Substanz auf einem Förderband, sind beispielsweise der initiale Feuchtegehalt, die Temperatur und Feuchte der Trocknungsluft und die Dauer des Trocknungsvorgangs relevant. Diese Parameter sind häufig zu einem großen Teil nicht oder lediglich eingeschränkt steuerbar, so dass der Trocknungsvorgang in der Regel keine gleichbleibenden Ergebnisse liefert und somit insbesondere der Feuchtegehalt in der Substanz am Ende des Trocknungsvorgangs stark variiert. Die existierenden Vorrichtungen und Verfahren zum Betrieb solcher Vorrichtungen bieten verschiedene Vorteile, jedoch sind weitere Verbesserungen wünschenswert.

Es ist daher eine Aufgabe der vorliegenden Erfindung, eine Lösung vorzuschlagen, welche einen oder mehrere der genannten Nachteile verringert oder beseitigt. Insbesondere ist es eine Aufgabe der vorliegenden Erfindung, eine Lösung vorzuschlagen, welche eine verbesserte Entmistung und/oder ein verbessertes Klima eines Stalls bereitstellt. Darüber hinaus ist es eine Aufgabe der vorliegenden Erfindung, eine Lösung vorzuschlagen, welche eine artgerechtere und/oder effizientere Tierhaltung ermöglicht.

Gemäß einem ersten Aspekt der vorliegenden Erfindung wird die eingangs genannte Aufgabe gelöst durch eine Trocknungsvorrichtung zur Trocknung einer Substanz, insbesondere zur Trocknung von Geflügelkot, umfassend eine Trocknungseinheit mit einer Fördervorrichtung, einen Trockensubstanzsensor und einen Ammoniaksensor.

Die Trocknungseinheit umfasst vorzugsweise einen Substanzeingang und einen Substanzausgang. Der Substanzeingang ist insbesondere angeordnet und ausgebildet, die ankommende zu trocknende Substanz, insbesondere Geflügelkot, der vorzugsweise aus einem Stall ab- und zu einer Trocknungsvorrichtung hin transportiert wurde, in die Trocknungseinheit hinein zu befördern bzw. zu leiten. Der Substanzausgang ist vorzugsweise angeordnet und ausgebildet, die getrocknete Substanz, insbesondere einen getrockneten Geflügelkot, aus der Trocknungseinheit hinaus zu befördern bzw. zu leiten. Eine vorteilhafte Ausbildung der Trocknungseinheit sieht ferner vor, dass diese zwei oder mehrere Substanzeingänge und/oder zwei oder mehrere Substanzausgänge umfasst. Die Trocknungseinheit umfasst demnach mindestens einen Substanzeingang und mindestens einen Substanzausgang. Zwischen dem mindestens einen Substanzeingang und dem mindestens einen Substanzausgang ist vorzugsweise ein Trocknungsbereich angeordnet.

Die zu trocknende Substanz wird vorzugsweise von dem einen Substanzeingang zu einer Fördervorrichtung gebracht, die die zu trocknende Substanz durch den Trocknungsbereich auf einer, vorzugsweise vorbestimmten, Bewegungsbahn durch den Trocknungsbereich hin zum mindestens einen Substanzausgang befördert. Vorzugsweise umfasst die Fördervorrichtung mindestens ein Förderband, welches vorzugsweise endlos ausgebildet ist. Ferner vorzugsweise umfasst die Fördervorrichtung zwei, drei oder mehrere Förderbänder, welche vorzugsweise vertikal übereinander angeordnet sind, wobei diese vorzugsweise in horizontaler Richtung und/oder in Förderrichtung teilweise versetzt sind, sodass die zu trocknende Substanz von einem oberen Förderband zu einem unteren Förderband gelangen kann. Vorzugsweise ist die Förderrichtung des oberen Förderbands entgegengesetzt zu der Förderrichtung des unteren Förderbands. Durch eine derartige Anordnung verfährt die zu trocknende Substanz vorzugsweise mehrmals durch einen Trocknungsbereich, in dem die Förderbänder vorzugsweise übereinander angeordnet sind. Die gesamte Strecke, die die Substanz durch die Trocknungseinheit zurücklegt, wird als Trocknungsstrecke bezeichnet.

Erfindungsgemäß umfasst die Trocknungsvorrichtung einen Trockensubstanzsensor. Der Trockensubstanzsensor kann den Anteil an trockener Substanz in einer Substanz ermitteln. Dieser Anteil wird aus als Trockensubstanzgehalt (TS-Gehalt) bezeichnet. Insbesondere Geflügelkot umfasst neben trockenen Substanzen, das heißt Feststoffen, in der Regel auch nicht trockene Substanzen, das heißt insbesondere Substanzen, die flüssig sind. Der flüssige Anteil ist in der Regel zu einem überwiegenden Anteil Wasser. Trockensubstanzsensoren können taktil und nicht taktil ausgebildet sein. Ferner können Trockensubstanzsensoren auch als Hybridvarianten mit taktilen und nicht taktilen Funktionen eingesetzt werden. Der Trockensubstanzsensor ist vorzugsweise in einem Bereich angrenzend an die Fördervorrichtung angeordnet. Ferner vorzugsweise ist der Trockensubstanzsensor derart angeordnet, dass dieser einen Kontakt zu der zu trocknenden Substanz herstellen kann.

Insbesondere ist es bevorzugt, dass der Trockensubstanzsensor in einem Bereich angrenzend an die Bewegungsbahn der Substanz zwischen dem Substanzeingang und dem Substanzausgang angeordnet ist. In einer besonders bevorzugten Ausführungsvariante umfasst die Trocknungsvorrichtung zwei oder mehrere Trockensubstanzsensoren.

Ferner umfasst die Trocknungsvorrichtung einen Ammoniaksensor. Der Ammoniaksensor misst insbesondere den Ammoniakgehalt der durchströmenden Luft, insbesondere der durchströmenden Luft durch die Trocknungseinheit. Damit lässt sich die Ammoniakausdünstung der zu trocknenden Substanz, insbesondere des Geflügelkots, unter anderem während des Trocknungsprozesses ermitteln. Darüber hinaus kann mit dem Ammoniaksensor ein Nachweis erbracht werden, wie hoch die tatsächliche Ammoniakemission des Stalls ist.

Ammoniak ist ein stark stechend riechendes, farbloses, wasserlösliches und insbesondere giftiges Gas, das lediglich zu einem geringen Anteil in der Stallluft vorhanden sein sollte. Um eine artgerechte Haltung der Tiere und ferner ein Wohlbefinden der Tiere zu gewährleisten, ist eine Reduktion des Ammoniakgehaltes in der Stallluft wünschenswert. Vorzugsweise ist der Ammoniaksensor innerhalb der Trocknungseinheit angeordnet. Ferner vorzugsweise umfasst die Trocknungsvorrichtung zwei oder mehrere Ammoniaksensoren, die an mehreren Stellen der Trocknungseinheit angeordnet sind. Eine derartige Anordnung von mehreren Ammoniaksensoren hat den Vorteil, dass über die Trocknungseinheit ein Mittelwert der Ammoniakbelastung erfasst werden kann. Ferner vorzugsweise sind die mehreren Ammoniaksensoren entlang der Bewegungsbahn der zu trocknenden Substanz innerhalb der Trocknungseinheit angeordnet.

Insbesondere ist es bevorzugt, dass ein Trockensubstanzsensor und/oder ein Ammoniaksensor in Bezug auf die Bewegungsbahn eine geringere Entfernung zum Substanzausgang als zum Substanzeingang aufweist. Insbesondere ist es bevorzugt, dass ein Trockensubstanzsensor und/oder ein Ammoniaksensor in einem Bereich der Bewegungsbahn angrenzend an den Substanzausgang angeordnet ist, wobei der Abstand zum Substanzausgang in Bezug auf die Bewegungsbahn kleiner als 50 %, kleiner als 40 %, kleiner als 30 %, kleiner als 20 %, kleiner als 10 %, oder kleiner als 5 % der Trocknungsstrecke beträgt.

Ferner vorzugsweise weist ein Trockensubstanzsensor und/oder ein Ammoniaksensor in Bezug auf die Bewegungsbahn eine geringere Entfernung zum Substanzeingang als zum Substanzausgang auf. Insbesondere ist es bevorzugt, dass ein Trockensubstanzsensor und/oder ein Ammoniaksensor in einem Bereich der Bewegungsbahn angrenzend an den Substanzeingang angeordnet ist, wobei der Abstand zum Substanzeingang in Bezug auf die Bewegungsbahn kleiner 51 %, kleiner als 40 %, kleiner als 30 %, kleiner als 20 %, kleiner als 10 %, oder kleiner als 5 % der Trocknungsstrecke beträgt.

Ferner vorzugsweise können mehrere Trockensubstanzsensoren und/oder mehrere Ammoniaksensoren äquidistant in Bezug auf die Bewegungsbahn angeordnet sein.

In einer bevorzugten Ausführungsvariante der Trocknungsvorrichtung ist vorgesehen, dass der Trockensubstanzsensor als NIR-Sensor und/oder Mikrowellensensor ausgebildet ist. NIR steht für Nahinfrarotspektroskopie, die eine physikalische Analysetechnik auf Basis der Spektroskopie im Bereich des kurzwelligen Infrarotlichts ist. Diese entspricht im Wesentlichen der Infrarotspektroskopie, die im mittel- und ferninfraroten Bereich (MIR und FIR) verwendet wird.

Der Trockensubstanzsensor weist vorzugsweise einen Messbereich von 0-85 % Restfeuchte und/oder eine Messgenauigkeit von weniger als einem Prozent absolut, insbesondere von ca. 0,5 % absolut auf. Ferner ist der Trockensubstanzsensor vorzugsweise geeignet für eine Temperatur des zu trockenen Gutes von 0 bis +80°C.

Gemäß einer weiteren bevorzugten Ausführungsvariante der Trocknungsvorrichtung ist vorgesehen, dass der Trockensubstanzsensor höhenbeweglich angeordnet ist. Dies bedeutet insbesondere, dass der Trockensubstanzsensor vertikal bzw. in einer Richtung orthogonal zur vorzugsweise flächigen Erstreckung der Fördereinheit, insbesondere eines Förderbands, beweglich angeordnet ist. Ein höhenbeweglicher Trockensubstanzsensor hat insbesondere den Vorteil, dass der Trockensubstanzgehalt stets in, vorzugsweise unmittelbarer, Nähe zur Oberfläche der zu trocknenden Substanz bestimmt werden kann. Vorzugsweise ist der Trockensubstanzsensor oberhalb der zu trocknenden Substanz angeordnet.

Gegenüber einer Anordnung eines Trockensubstanzsensors unterhalb der Fördervorrichtung und/oder einer von der zu trocknenden Substanz deutlich beabstandeten Anordnung (beispielsweise weit oberhalb der Substanz), hat die vorgesehene höhenbewegliche Anordnung den Vorteil, dass die Position des Trockensubstanzsensors auch auf eine variierende Substanzhöhe angepasst werden kann, so dass damit aussagekräftigere und/oder zuverlässigere Messwerte erzeugt werden können. Es ist insbesondere bevorzugt, dass die Höhenbeweglichkeit in Abhängigkeit der Höhe der zu trocknenden Substanz auf der Fördervorrichtung erfolgt.

Gemäß einer weiteren bevorzugten Ausführungsvariante der Trocknungsvorrichtung ist vorgesehen, dass der Trockensubstanzsensor derart angeordnet und ausgebildet ist, dass sich der Trockensubstanzsensor auch bei unterschiedlichen Substanzhöhen auf der Fördervorrichtung im Wesentlichen unmittelbar auf der Substanz befindet. Auf der Substanz bedeutet insbesondere, dass sich der Trockensubstanzsensor auf einer oder in, vorzugsweise unmittelbarer, Nähe zur Oberfläche der zu trocknenden Substanz befindet.

Gemäß einer weiteren bevorzugten Ausführungsvariante der Trocknungsvorrichtung ist vorgesehen, dass der Trockensubstanzsensor an einer Aufhängevorrichtung angeordnet ist, die vorzugsweise oberhalb der Fördervorrichtung angeordnet ist. Durch den Trockensubstanzsensor, der an einer Aufhängevorrichtung angeordnet ist, kann gewährleistet oder verbessert werden, dass der Trockensubstanzsensor in einer definierten und/oder einer variierenden Höhe über der Fördervorrichtung angeordnet ist. Insbesondere kann durch eine Aufhängevorrichtung gewährleistet werden, dass sich der Trockensubstanzsensor auch bei unterschiedlichen Substanzhöhen auf der Fördervorrichtung im Wesentlichen stets unmittelbar auf der Substanz bzw. auf der Oberfläche der Substanz befindet.

Gemäß einer besonders bevorzugten Ausführungsvariante der Trocknungsvorrichtung ist vorgesehen, dass die Aufhängevorrichtung einen Schlitten umfasst und/oder dass die Aufhängevorrichtung ein Führungselement umfasst, das vorzugsweise an seinem ersten und/oder seinem zweiten Ende drehbar gelagert ist. Der Trockensubstanzsensor ist vorzugsweise an dem Schlitten angeordnet. Das Führungselement ist vorzugsweise im Wesentlichen stabförmig. Vorzugsweise ist das Führungselement mit einem Ende an einem statischen Bauteil der Trocknungsvorrichtung und mit dem anderen Ende an dem Schlitten angeordnet.

Das Vorsehen eines Schlittens hat den Vorteil, dass auch bei einer in der Regel hohen Dichte der zu trocknenden Substanz in Stallvorrichtungen, der Schlitten, vorzugsweise mit Trockensubstanzsensor, im Wesentlichen auf der Oberfläche der Substanz angeordnet ist. Der Schlitten weist vorzugsweise eine flächige Erstreckung auf, mit einer Unterseite und einer Oberseite. Orthogonal zu der flächigen Erstreckung weist der Schlitten an mindestens einer seiner Kanten vorzugsweise eine, zwei oder mehrere Seitenwände auf, die vorzugsweise eine flächige Erstreckung orthogonal zur Ober- und/oder Unterseite aufweisen. Insbesondere ist es bevorzugt, dass der Schlitten eine Seitenwand an der Kante aufweist, deren flächige Erstreckung geneigt, beispielsweise zwischen 5 und 90°, insbesondere zwischen 30 und 60°, beispielsweise um 45°, zur Förderrichtung ausgerichtet ist.

Vorzugsweise sind die Seitenwände vom Mittelpunkt des Schlittens nach außen geneigt. Durch diese geneigten Seitenwände wird ferner gewährleistet oder verbessert, dass der Schlitten auf der Oberfläche der zu trocknenden Substanz angeordnet ist und auch dort verbleibt, wenn die Fördereinheit mit der zu trocknenden Substanz in Förderrichtung relativ zum Schlitten bewegt wird. Die Unterfläche des Schlittens ist vorzugsweise der zu trocknenden Substanz zugewandt. Die Oberfläche des Schlittens ist vorzugsweise der zu trocknenden Substanz abgewandt.

Vorzugsweise ist der Trockensubstanzsensor auf der Oberfläche des Schlittens angeordnet. Darüber hinaus kann der Schlitten an der Stelle, an der der Trockensubstanzsensor an dem Schlitten angeordnet ist, eine Aussparung aufweisen, sodass der Trockensubstanzsensor in einem unmittelbaren Kontakt mit der zu trocknenden Substanz auf der Unterseite des Schlittens gelangen kann.

Eine derartige Aufhängevorrichtung mit einem Führungselement und einem Schlitten ermöglicht die kostengünstige und insbesondere sichere Anordnung eines Trockensubstanzsensors auf der Oberfläche der zu trocknenden Substanz, ohne notwendigerweise eine komplexe Steuervorrichtung, beispielsweise mit einem Höhensensor und/oder einer automatischen Einstellung der Höhe des Trockensubstanzsensors, vorzusehen. Die Rotationsachsen des Führungselements der Aufhängevorrichtung und/oder des Schlittens sind vorzugsweise im Wesentlichen parallel und/oder im Wesentlichen orthogonal zur Fördervorrichtung.

Gemäß einer weiteren besonders bevorzugten Ausführungsvariante der Trocknungsvorrichtung ist vorgesehen, dass die Trocknungsvorrichtung einen oder mehrere Sensoren aufweist, die ausgebildet sind einen oder mehrere weitere Substanzparameter, wie beispielsweise den Stickstoff- und/oder Stickoxid- und/oder Phosphor- und/oder Proteingehalt der Substanz, zu ermitteln. Vorzugsweise ist der Trockensubstanzsensor angeordnet und ausgebildet, einen oder mehrere weitere Substanzparameter, wie beispielsweise den Stickstoff- und/oder Stickoxid- und/oder Phosphor- und/oder Proteingehalt der Substanz, zu ermitteln.

Gemäß einer weiteren besonders bevorzugten Ausführungsvariante der Trocknungsvorrichtung ist vorgesehen, dass die Trocknungsvorrichtung einen Volumenstromsensor umfasst, der ausgebildet und angeordnet ist zur Messung eines die Trocknungsvorrichtung durchströmenden Fluides, wie beispielsweise Luft. In einer bevorzugten Kombination mit dem Ammoniaksensor lässt sich eine sogenannte Ammoniakfracht ermitteln, welche in einer Stallbilanz zusammengefasst und ausgewertet werden kann. Die Ammoniakfracht ist beispielsweise die pro Zeiteinheit aus dem Stall beförderte Ammoniakmenge. Ferner vorzugsweise umfasst die Trocknungsvorrichtung mindestens eine Ventilationseinrichtung, um Luft innerhalb der Trocknungsvorrichtung und/oder im Austausch mit der Umgebung zirkulieren zu können.

Gemäß einer weiteren besonders bevorzugten Ausführungsvariante der Trocknungsvorrichtung ist vorgesehen, dass der Trockensubstanzsensor und/oder der Ammoniaksensor und/oder der Volumenstromsensor angeordnet und ausgebildet ist bzw. sind, Sensordaten an eine Steuerungseinheit zu übermitteln. Die Steuerungseinheit ist vorzugsweise derart ausgebildet und angeordnet, die Trocknungsvorrichtung oder ein Trocknungssystem derart zu steuern, dass die gewünschten Zustände, beispielsweise in Bezug auf den Ammoniakgehalt und/oder den Trockensubstanzgehalt in der zu trocknenden Substanz, zu beeinflussen.

Gemäß einer weiteren besonders bevorzugten Ausführungsvariante der Trocknungsvorrichtung ist vorgesehen, dass der Steuerungsparameter einen oder mehrere der folgenden Parameter umfasst, für die Trocknung der Substanz erforderliche Zeit, maximale und/oder minimale Höhe der Substanz auf der Trocknungseinheit und/oder einem Transportsystem, Menge und/oder Volumen eines rezirkulierten und/oder abventilierten Fluids in der Trocknungsvorrichtung, Geschwindigkeit der Fördervorrichtung und/oder der Kottransporteinheiten, Start- und/oder Stoppsignal für die Fördervorrichtung und/oder die Kottransporteinheit.

Gemäß einer weiteren besonders bevorzugten Ausführungsvariante der Trocknungsvorrichtung ist vorgesehen, dass die Steuerungseinheit ausgebildet ist, aus den Sensordaten eine Bilanz und/oder Gesamtmenge der während eines vorgegebenen Zeitraums emittierten Stoffe, insbesondere Ammoniak und/oder Stickstoff und/oder Phosphat und/oder rezirkuliertes Fluid und/oder abventiliertes Fluid, zu ermitteln. Somit kann für den Stall eine Bilanz, die auch als Gesamtbilanz oder Stallbilanz bezeichnet werden kann, erstellt werden, die insbesondere aufzeigt, welche Stoffe, insbesondere unerwünschte Stoffe beziehungsweise schädigende Stoffe, emittiert werden bzw. wurden. Vorzugsweise ist die Steuerungseinheit angeordnet und ausgebildet, auf Basis der Stallbilanz beziehungsweise der im Vorherigen genannten Sensordaten Einfluss auf die unterschiedlichen Parameter der Trocknungsvorrichtung einzuwirken, sodass eine Stallbilanz im Rahmen von gewünschten Werten ausfällt.

Ferner vorzugsweise ist die Steuerungseinheit ausgebildet, mindestens einen oder mehrere der folgenden Parameter zu empfangen und/oder auszuwerten: eine Belastung der Fördervorrichtung und/oder eine Belastung vom in einem Stall angeordneten Fördervorrichtung in und/oder Umgebungsparameter in der Trocknungsvorrichtung und oder in einem Nutztierstall, wie beispielsweise Ammoniakgehalt der Luft und/oder Temperatur und/oder Luftfeuchtigkeit. Ferner ist bevorzugt, dass die Steuerungseinheit ausgebildet ist, mindestens ein Steuersignal zu generieren, mit dem die Trocknungsvorrichtung und/oder Kottransporteinheiten in einem Nutztierstall gesteuert werden können.

Darüber hinaus ist es bevorzugt, dass die Steuerungseinheit selbstlernend ausgebildet ist und/oder ausgebildet ist, aus früheren Daten Prognosen zu generieren. Auf diese Weise können beispielsweise Entmistungsintervalle für die Zukunft abgeleitet werden. Bevorzugt ist ferner, dass die Steuerungseinheit ausgebildet ist, eine vollautomatische Entmistung eines Nutztierstalls durchzuführen.

Gemäß einer weiteren bevorzugten Ausführungsvariante umfasst die Trocknungsvorrichtung einen Beladungssensor.

Ein Beladungssensor dient vorzugsweise dazu, die Beladung der Fördereinheit mit zu trocknender Substanz zu ermitteln. Vorzugsweise können die Geschwindigkeit und/oder die Geschwindigkeit, mit der zu trocknende Substanz in den Substanzeingang eingeführt wird, und/oder die Trocknungsdauer und/oder weitere für die Entmistung und/oder den Trocknungsvorgang relevante Parameter in Abhängigkeit von der durch den Beladungssensor ermittelten Beladung gesteuert werden.

Gemäß einem weiteren Aspekt der vorliegenden Erfindung wird die eingangs genannte Aufgabe gelöst durch ein Steuerungssystem für eine Trocknungsvorrichtung zum Trocknen einer Substanz, insbesondere zum Trocknen von Geflügelkot, vorzugsweise für eine Trocknungsvorrichtung nach mindestens einer der im Vorherigen beschriebenen Ausführungsvarianten, umfassend eine Steuerungseinheit, die angeordnet und ausgebildet ist, Sensordaten von einem oder mehreren Sensoren, insbesondere von einem Trockensubstanzsensor und/oder von einem Ammoniaksensor und/oder von einem Volumenstromsensor und/oder von einem Beladungssensor einer Kottransportvorrichtung, zu empfangen und aus den Sensordaten einen oder mehrere Steuerungsparameter für den Betrieb einer Trocknungsvorrichtung und/oder einer Kottransportvorrichtung abzuleiten und den oder die Steuerungsparameter an eine Trocknungsvorrichtung und/oder eine oder mehrere Kottransportvorrichtungen zu senden. Die Steuerungsparameter umfassen vorzugsweise einen oder mehrere der folgenden Parameter, für die Trocknung der Substanz erforderliche Zeit, maximale und/oder minimale Höhe der Substanz auf und/oder in der Trocknungseinheit und/oder einem Transportsystem, Menge und/oder Volumen eine rezirkulierten und/oder abventilierten Fluids in der Trocknungsvorrichtung, Geschwindigkeit der Fördervorrichtung und/oder der Kottransporteinheiten, Start- und/oder Stoppsignal für die Fördervorrichtung und/oder die Kottransporteinheiten. Insbesondere können auf Basis dieser Steuerungseinheiten die Trocknungsvorrichtung und/oder eine oder mehrere Kottransportvorrichtungen derart gesteuert werden, dass der Ammoniakgehalt in der Luft des Stalls und/oder der Trockensubstanzgehalt der zu trocknenden Substanz, insbesondere am Substanzausgang, einen oder mehrere gewünschte Werte erreicht.

In einer bevorzugten Ausführungsvariante des Steuerungssystem ist vorgesehen, dass die Steuerungseinheit angeordnet und ausgebildet ist, bei der Ermittlung des oder der Steuerungsparameter eine oder mehrere Randbedingungen zu berücksichtigen, wie beispielsweise eine Totzeit der Trocknungsvorrichtung und/oder einer oder mehrerer Kottransportvorrichtungen während eines Betriebs von Eiersammel- und/oder Eierfördervorrichtungen und/oder eine oder mehrere Benutzervorgaben. In einer weiteren vorteilhaften Ausführungsvariante weist das Steuerungssystem eine Benutzerschnittstelle zur Eingabe von Benutzervorgaben auf. Benutzervorgaben können vorliegend beispielsweise der Trockensubstanzanteil und/oder der Ammoniakanteil beziehungsweise die Ammoniakmenge in der Abluft des Stalls beziehungsweise des Trocknungsbereiches beziehungsweise der Trocknungsvorrichtung sein.

Gemäß einem weiteren Aspekt der Erfindung wird die eingangs genannte Aufgabe gelöst durch ein Trocknungssystem, umfassend eine Trocknungsvorrichtung nach mindestens einer der im Vorherigen beschriebenen Ausführungsvarianten, und ein Steuerungssystem nach mindestens einer der im Vorherigen beschriebenen Ausführungsvarianten. In einer besonders bevorzugten Ausführungsvariante des Trocknungssystems ist vorgesehen, dass die Trocknungsvorrichtung außerhalb eines Geflügelstalls angeordnet ist. Durch die Anordnung der Trocknungsvorrichtung außerhalb des Geflügelstalls besteht die Möglichkeit, dass die Nebenprodukte, wie Ammoniak und/oder Geflügelkot, schnell aus dem Geflügelstall entfernt werden und die in dem Geflügelstall befindlichen Tiere somit nicht mehr oder lediglich in vermindertem Maße den Belastungen der Nebenstoffe ausgesetzt sind.

Gemäß einem weiteren Aspekt der vorliegenden Erfindung wird die eingangs genannte Aufgabe gelöst durch ein Geflügelhaltungssystem, umfassend eine Trocknungsvorrichtung nach mindestens einer der im Vorherigen beschriebenen Ausführungsvarianten und/oder ein Steuerungssystem nach mindestens einer der im Vorherigen beschriebenen Ausführungsvarianten und/oder ein Trocknungssystem nach mindestens einer der im Vorherigen beschriebenen Ausführungsvarianten und/oder eine oder mehrere Kottransportvorrichtungen. In einer besonders bevorzugten Ausführungsvariante des Geflügelhaltungssystems, ist die Trocknungsvorrichtung außerhalb eines Geflügelstalls angeordnet.

Gemäß einem weiteren Aspekt der Erfindung wird die eingangs genannte Aufgabe gelöst durch ein Verfahren zur Trocknung einer Substanz, umfassend Bereitstellen einer Trocknungseinheit mit einer Fördervorrichtung, Ermitteln eines Trockensubstanzgehalts und/oder einer Ammoniakemission in der Fördervorrichtung.

Das erfindungsgemäße Verfahren und seine möglichen Fortbildungen weisen Merkmale beziehungsweise Verfahrensschritte auf, die sie insbesondere dafür geeignet machen, für eine erfindungsgemäße Trocknungsvorrichtung, ein Steuerungssystem, ein Trocknungssystem und/oder ein Geflügelhaltungssystem und ihre jeweiligen Fortbildungen verwendet zu werden. Für weitere Vorteile, Ausführungsvarianten und Ausführungsdetails dieser weiteren Aspekte und ihrer möglichen Fortbildungen wird auch auf die zuvor erfolgte Beschreibung zu den entsprechenden Merkmalen und Fortbildungen der Trocknungsvorrichtung verwiesen.

Bevorzugte Ausführungsformen der Erfindung werden beispielhaft anhand der beiliegenden Figuren erläutert. Es zeigen:
- Fig. 1:: eine erste beispielhafte Ausführungsform einer Trocknungsvorrichtung;
- Fig. 2:: eine beispielhafte Ausführungsform einer Aufhängevorrichtung mit Schlitten;
- Figur 3:: eine weitere beispielhafte Ausführungsform einer Trocknungsvorrichtung;
- Figur 4:: eine beispielhafte Ausführungsform eines Geflügelhaltungssystems.

In den Figuren sind gleiche oder im Wesentlichen funktionsgleiche bzw. -ähnliche Elemente mit den gleichen Bezugszeichen bezeichnet.

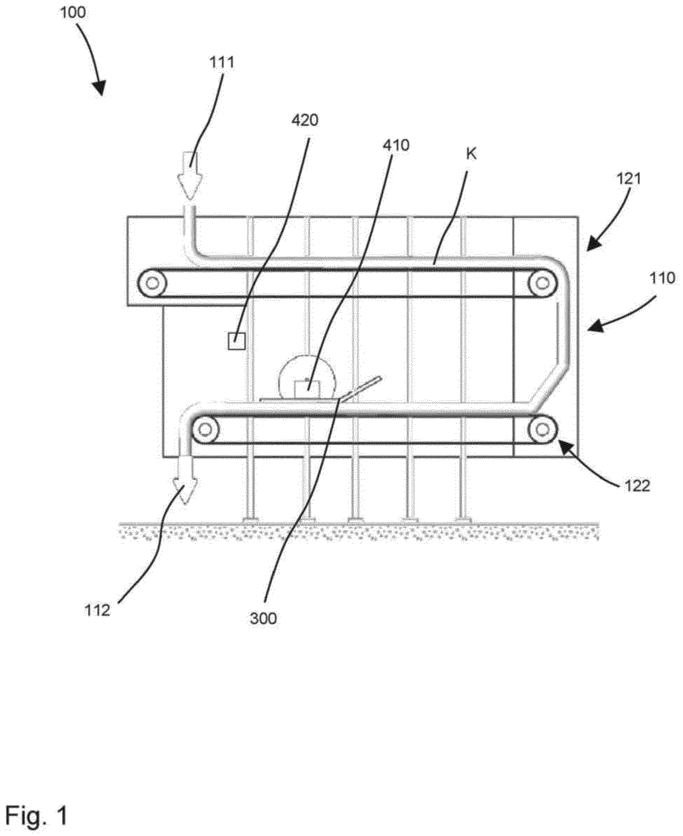

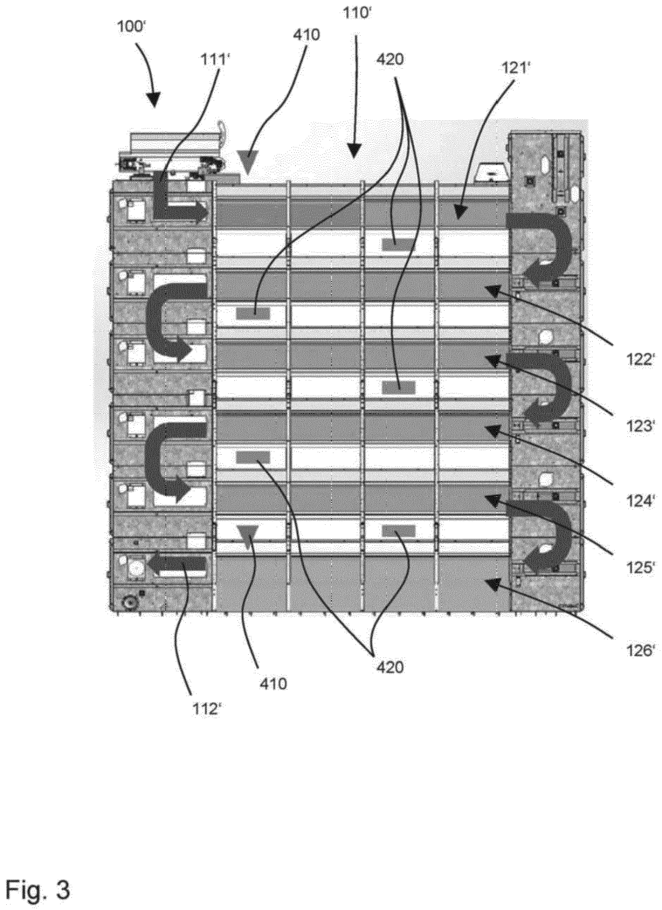

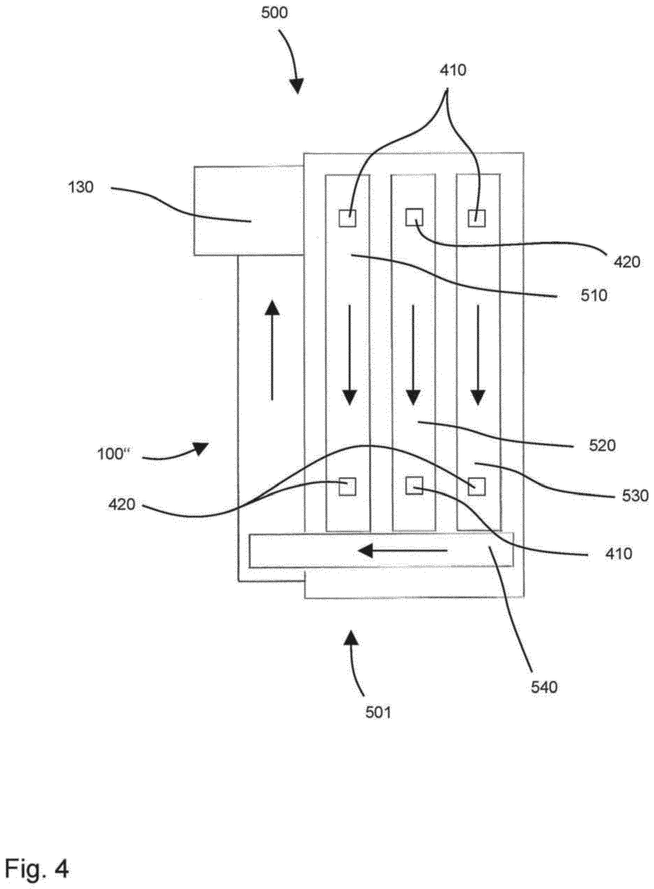

Figur 4 zeigt ein Geflügelhaltungssystem 500 mit einem Geflügelstall 501, in dem mehrere Kottransporteinheiten 510, 520, 530, 540 angeordnet sind. Die Förderrichtungen der Kottransporteinheiten 510, 520, 530, 540 sind mit Pfeilen gekennzeichnet. Die drei Kottransporteinheiten 510, 520, 530 sind parallel angeordnet und dienen dazu, gut von in einem Stall in der ebenfalls in Reihen angeordneten Tieraufenthaltsbereichen (nicht dargestellt) abzutransportieren. Am Anfang und am Ende der drei Kottransporteinheiten 510, 520, 530 sind jeweils Trockensubstanzsensoren 410 und Ammoniaksensoren 420 angeordnet. Die drei Kottransporteinheiten 510, 520, 530 fördern den Kot auf eine weitere, zu diesen drei Kottransporteinheiten 510, 520, 530 quer angeordnete Kottransporteinheit 540. Diese Kottransporteinheit 540 dient dazu, den gesamten im Geflügelstall 501 angefallenen Kot zu einer außerhalb des Geflügelstalls 501 angeordnete Trocknungsvorrichtung 100" zu verbringen. Nach dem der Kot die Trocknungsvorrichtung 100" im Wesentlichen in der in Figur 1 mit einem Pfeil gekennzeichneten Förderrichtung verlassen hat, kann der getrocknete Kot in einer Zwischenlagerungsvorrichtung 130 zwischengelagert werden oder auch direkt verpackt und/oder weitertransportiert werden. Die Trocknungsvorrichtung 100" kann beispielsweise gemäß den in den Figuren 1 und 3 dargestellten beispielhaften Ausführungsformen von Trocknungsvorrichtungen 100,100' ausgebildet sein.

In den Figuren 1 und 3 sind beispielhafte Ausführungsformen von Trocknungsvorrichtungen 100, 100' dargestellt. Die in Figur 1 gezeigte Trocknungsvorrichtung 100 weist eine Trocknungseinheit 110 mit zwei Fördervorrichtungen 121, 122, die den Kot K von einem Substanzeingang 111 entlang einer Bewegungsbahn zum Substanzausgang 112 fördern. Die Förderrichtungen der beiden Fördervorrichtungen 121,122 sind dabei entgegengesetzt.

Die in Figur 3 dargestellte Trocknungsvorrichtung 100' weist eine Fördereinheit 110' mit sechs Fördervorrichtungen 121', 122', 123', 124', 125', 126' auf, die den Kot von einem Substanzeingang 111' zu einem Substanzausgang 112' fördern. Die Förderrichtungen von jeweils zwei benachbarten Fördervorrichtungen 121', 122', 123', 124', 125', 126' verlaufen vorzugsweise entgegengesetzt. Wie in Figur 3 mit den Pfeilen dargestellt, verläuft die Bewegungsbahn des Kots mäanderförmig.

In der Fördervorrichtung 100' gemäß Figur 3 sind in der Nähe des Substanzeingangs 111' und in der Nähe des Substanzausgangs 112' zwei Trockensubstanzsensoren 410 angeordnet. Ferner sind vier entlang der Bewegungsbahn zwischen den Trockensubstanzsensoren 410 angeordnete Feuchtigkeits- und Temperaturfühler 420' vorgesehen. In der Fördervorrichtung 100 gemäß Figur 1 sind ein Trockensubstanzsensor 410 und ein Feuchtigkeits- und Temperaturfühler 420' dargestellt. In beiden Trocknungsvorrichtungen 100,100' sind die Trockensubstanzsensoren 410 vorzugsweise auf einem Schlitten 300 (in Figur 3 zu erkennen) angeordnet.

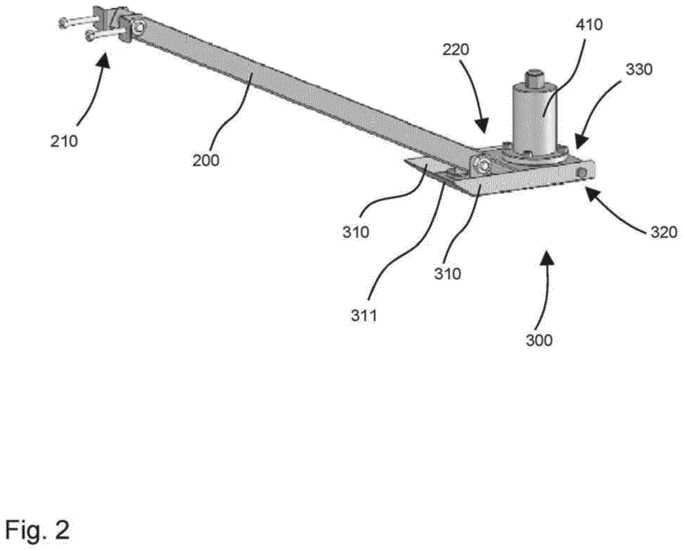

Figur 2 zeigt diesen Schlitten 300 mit seiner Aufhängevorrichtung 200 im Detail. Die Aufhängevorrichtung 200 weist an ihrem ersten Ende 210 und an ihrem zweiten Ende 220 Befestigungselemente auf, mit denen am zweiten Ende 220 der Schlittens 300 schwenkbeweglich befestigt werden kann. Am ersten Ende 210 kann die Aufhängevorrichtung 200 an der Trocknungsvorrichtung schwenkbeweglich befestigt werden. Die Rotationsachsen für die Schwenkbewegung der Aufhängevorrichtung 200 an ihrem ersten Ende 210 und an ihrem zweiten Ende 220 sind vorzugsweise im Wesentlichen horizontal und ferner im Wesentlichen orthogonal zur Förderrichtung.

Auf einer Oberfläche 330 des Schlittens 300 ist ein Trockensubstanzsensor 410 angeordnet. Der Trockensubstanzsensor 410 reicht vorzugsweise durch eine Ausnehmung im Schlitten 330 hindurch, sodass ein Sensorelement des Trockensubstanzsensors 410 vorzugsweise auf der Unterfläche 320 des Schlittens 300 in unmittelbarer Nähe zu oder auf einer Oberfläche des zu trocknenden Kots angeordnet sein kann. Die parallel zur Förderrichtung ausgerichteten Seitenwände 310 des Schlittens 300 sind orthogonal zur Unterfläche 320 geneigt. Eine weitere Seitenwand 311 ist unter einem Winkel von ca. 45° zur Förderrichtung geneigt.

Durch die Aufhängevorrichtung 200 wird eine höhenbewegliche Befestigung des Trockensubstanzsensors 410 ermöglicht, sodass der Trockensubstanzsensor 410 einfach und ohne großen erforderlichen Steuerungsaufwand der Beladung der Fördervorrichtungen mit Kot als zu trocknender Substanz folgen kann. Der Schlitten 300, insbesondere mit seiner vorderen, zur Förderrichtung geneigten Seitenwand 311, dient dazu, auf dem Kot entlang zu gleiten und ebenfalls sicherzustellen, dass sich der Trockensubstanzsensor 410 auf oder in unmittelbarer Nähe zu der Oberfläche des Kots befindet.

Durch die Ermittlung des Trockensubstanzgehalts mittels des Trockensubstanzsensors 410 und die gleichzeitige Ermittlung der Ammoniakausdünstung mit einem Ammoniaksensor 420 kann zum einen der Trocknungsprozess und/oder die Qualität des am Ende des Trocknungsprozesses erhaltenen getrockneten Guts verbessert werden.

Ferner können die über die Sensoren gewonnenen Daten der Erstellung einer Stallbilanz und/oder der Ermittlung einer Gesamtmenge von Emissionen des Stalls dienen. Auf diese Weise können beispielsweise Entmistungsintervalle so gesteuert werden, dass Emissionswerte im Stall bestimmte Grenzwerte nicht überschreiten, was das Tierwohl deutlich verbessern kann. Gleichzeitig kann der Trocknungsprozess auf die im zu trocknenden Gut enthaltene Feuchte und/oder Randparameter, wie beispielsweise Umgebungstemperaturen und/oder Luftfeuchte, abgestimmt werden, so dass ein Endprodukt mit hoher, gleichbleibender Qualität in einem effizienten Trocknungsprozess, und damit auch mit effizientem Ressourceneinsatz, ermöglicht wird.

## Patentansprüche

1. Trocknungsvorrichtung zur Trocknung einer Substanz, insbesondere zur Trocknung von Geflügelkot, umfassend
eine Trocknungseinheit mit einer Fördervorrichtung,
einen Trockensubstanzsensor und einen Ammoniaksensor.

2. Trocknungsvorrichtung nach mindestens einem der vorhergehenden Ansprüche,
**dadurch gekennzeichnet, dass** der Trockensubstanzsensor als NIR-Sensor und/oder Mikrowellensensor ausgebildet ist.

3. Trocknungsvorrichtung nach mindestens einem der vorhergehenden Ansprüche,
**dadurch gekennzeichnet, dass** der Trockensubstanzsensor höhenbeweglich angeordnet ist.

4. Trocknungsvorrichtung nach mindestens einem der vorhergehenden Ansprüche,
**dadurch gekennzeichnet, dass** der Trockensubstanzsensor derart angeordnet und ausgebildet ist, dass sich der Trockensubstanzsensor auch bei unterschiedlichen Substanzhöhen auf der Fördervorrichtung im Wesentlichen unmittelbar auf der Substanz befindet.

5. Trocknungsvorrichtung nach mindestens einem der vorhergehenden Ansprüche,
**dadurch gekennzeichnet, dass** der Trockensubstanzsensor an einer Aufhängevorrichtung angeordnet ist, die vorzugsweise oberhalb der Fördervorrichtung angeordnet ist.

6. Trocknungsvorrichtung nach mindestens einem der vorhergehenden Ansprüche,
**dadurch gekennzeichnet, dass** die Aufhängevorrichtung einen Schlitten umfasst
und/oder
**dadurch gekennzeichnet, dass** die Aufhängevorrichtung ein Führungselement umfasst, das vorzugsweise an seinem ersten und/oder seinem zweiten Ende drehbar gelagert ist.

7. Trocknungsvorrichtung nach mindestens einem der vorhergehenden Ansprüche,
**dadurch gekennzeichnet, dass** der Trockensubstanzsensor angeordnet und ausgebildet ist, einen oder mehrere weitere Substanzparameter, wie beispielsweise den Stickstoff- und/oder Phosphorgehalt der Substanz, zu ermitteln.

8. Trocknungsvorrichtung nach mindestens einem der vorhergehenden Ansprüche,
umfassend einen Volumenstromsensor, der ausgebildet und angeordnet ist zur Messung eines die Trocknungsvorrichtung durch strömenden Fluids, wie beispielsweise Luft.

9. Trocknungsvorrichtung nach mindestens einem der vorhergehenden Ansprüche,
**dadurch gekennzeichnet, dass** der Trockensubstanzsensor und/oder der Ammoniaksensor und/oder Volumenstromsensor angeordnet und ausgebildet ist bzw. sind, Sensordaten an eine Steuerungseinheit zu übermitteln.

10. Trocknungsvorrichtung nach mindestens einem der vorhergehenden Ansprüche,
**dadurch gekennzeichnet, dass** der Steuerungsparameter einen oder mehrere der folgenden Parameter umfasst:
- für die Trocknung der Substanz erforderliche Zeit,
- maximale und/oder minimale Höhe der Substanz auf der Trocknungseinheit und/oder einem Transportsystem,
- Menge und/oder Volumen eines rezirkulierten Fluid und/oder abventilierten Fluids in der Trocknungsvorrichtung,
- Geschwindigkeit der Fördervorrichtung und/oder der Kottransporteinheit(en),
- Start- und/oder Stoppsignal für die Fördervorrichtung und/oder die Kottransporteinheit(en).

11. Trocknungsvorrichtung nach mindestens einem der vorhergehenden Ansprüche,
**dadurch gekennzeichnet, dass** die Steuerungseinheit ausgebildet ist, aus den Sensordaten eine Bilanz und/oder Gesamtmenge der während eines vorgegebenen Zeitraums emittierten Stoffe, insbesondere Ammoniak und oder Stickstoff und oder Phosphat und oder rezirkuliertes und/oder abventiliertes Fluid, zu ermitteln.

12. Steuerungssystem für eine Trocknungsvorrichtung zum Trocknen einer Substanz, insbesondere zum Trocknen von Geflügelkot, vorzugsweise für eine Trocknungsvorrichtung nach mindestens einem der vorhergehenden Ansprüche,
umfassend eine Steuerungseinheit, die angeordnet und ausgebildet ist, Sensordaten von einem oder mehreren Sensoren, insbesondere von einem Trockensubstanzsensor und/oder von einem Ammoniaksensor und/oder von einem Volumenstromsensor und/oder von einem Beladungssensor einer Kottransportvorrichtung, zu empfangen und aus den Sensordaten einen oder mehrere Steuerungsparameter für den Betrieb einer Trocknungsvorrichtung und/oder einer Kottransportvorrichtung abzuleiten und den oder die Steuerungsparameter an eine Trocknungsvorrichtung und/oder eine oder mehrere Kottransportvorrichtungen zu senden.

13. Trocknungssystem, umfassend eine Trocknungsvorrichtung nach mindestens einem der vorhergehenden Ansprüche, und ein Steuerungssystem nach mindestens einem der vorhergehenden Ansprüche.

14. Geflügelhaltungssystem, umfassend eine Trocknungsvorrichtung nach mindestens einem der vorhergehenden Ansprüche und/oder ein Steuerungssystem nach mindestens einem der vorhergehenden Ansprüche und/oder ein Trocknungssystem nach mindestens einem der vorhergehenden Ansprüche und/oder eine oder mehrere Kottransportvorrichtungen.

15. Verfahren zur Trocknung einer Substanz, umfassend
- Bereitstellen einer Trocknungseinheit mit einer Fördervorrichtung,
- Ermitteln eines Trockensubstanzgehalts und/oder einer Ammoniakemission in der Fördervorrichtung.
